# EUROPEAN PATENT SPECIFICATION

(11) **EP 0 549 062 B1**
(45) Date of publication and mention of the grant of the patent: **17.03.1999**
(21) Application number: 92203992.0
(22) Date of filing: 17.12.1992
(51) Int. Cl.: C12N 15/62, C12N 15/80, C12N 15/55, C12N 15/31

(54) **A eukaryotic expression system**
Eukaryotisches Expressionssystem
Système d'expression eucaryote

(30) Priority: 23.12.1991 EP 91203400
(43) Date of publication of application: 30.06.1993
(73) Proprietor: GIST-BROCADES N.V., NL-2600 MA Delft (NL)
(72) Inventor: Bovenberg, Roelof Ary Lans, NL-3062 ZD Rotterdam (NL); Vollebregt, Adrianus Wilhelmus Hermanus, NL-2671 WZ Naaldwijk (NL); Van Solingen, Pieter, NL-2671 VZ Naaldwijk (NL)
(74) Representative: Visser-Luirink, Gesina, Dr.

(56) References cited:
- EP-A- 0 354 624
- EP-A- 0 420 358

## Description

This application relates to a gene expression and protein secretion system for eukaryotic cells, particularly fungal cells.

Commercial production of proteins is a main objective of industrial biotechnology. To that end a range of organisms are used, most of them being microorganisms, especially bacteria of the species Escherichia, Bacillus, Actinomycetes; yeasts of the species Saccharomyces and Kluyveromyces; and filamentous fungi of the species Aspergillus and Trichoderma. The ability to introduce and express novel DNA sequences into living cells has created many new possibilities for protein production by micro-organisms.

The development of reliable gene transfer systems for filamentous fungi has greatly stimulated interests for application of these microorganisms in commercial protein production processes. These interests are explained by the following facts:
1) by nature, filamentous fungi are able to synthesize and secrete large amounts of proteins;
2) several fungi have a long record of industrial use and are generally recognized as save (GRAS) production organisms;
3) considerable knowledge and experience is available on large scale fermentation and processing of fungal cultures;
4) the structure of heterologous proteins produced in filament-ous fungi closely mimics (or is identical to) the authentic structure of these proteins with regard to termini, modification and folding. This is often not achieved by using bacterial or yeast cells as hosts for heterologous gene expression;
5) the stability of transformed rDNA in fungal strains.

Despite all these favourable characteristics only a limited number of filamentous fungus species are in use in protein production processes. Furthermore, expression of heterologous proteins in these filamentous fungus species is generally not as efficient as expression of homologous proteins.

Thus, systems which allow for efficient gene expression and protein secretion and which extend the repertoire of filamentous fungus hosts for protein production are in great demand, particularly for application to Penicillium chrysogenum. P. chrysogenum is the fungus used world-wide for over 40 years for commercial production of penicillin. As a consequence great experience has been obtained in fermentation of P. chrysogenum. Moreover, non-penicillin producing mutants of P. chrysogenum are available which avoid the problem of penicillin contamination of the produced protein. Up to now, such an efficient gene expression and protein secretion system has not been provided for.

Filamentous fungus species like Aspergillus niger, Aspergillus oryzae, Mucor miehei and Trichoderma reesei are used in the industrial production of enzymes, e.g. for use in the food industry. See for example Strijkert (Antonie van Leeuwenhoek 53 (1987), 357-362) and Unkles (in: Molecular and Genetic Aspects of Nitrate Assimilation, Wray, J.L. and Kinghorn, J.R. (eds), 1989, 341-363, Oxford Science Publications, Oxford, UK).

Aspergillus niger (D. Cullen et al., Biotechnology 5 (1987), 369-376; A. Haarki et al., Biotechnology 7 (1989), 596-600; and EP-A-420358), Aspergillus nidulans (G.L. Gray et al., Gene 48 (1986), 41-53; and D.I. Gwynne et al., Biotechnology 5 (1987), 713-719), and Aspergillus oryzae (T. Christensen et al., Biotechnology 6 (1988), 1419-1422) have also been used for expression of various heterologous proteins for use in food and detergents and in pharmaceutical industry.

The expression systems used employ sequences for initiation and termination of transcription and translation, and sequences for processing and secretion of the expressed protein obtained from the A. niger glucoamylase (gla) gene, the T. reesei cellobiohydrolase (cbhI) gene, the A. oryzae α-amylase gene and the A. nidulans alcoholdehydrogenase (alcA) gene (D.I. Gwynne et al., supra; A. Haarki et al., supra; and T. Christensen et al., supra).

The present invention provides an efficient gene expression and protein secretion system for eukaryotic microorganisms, especially for filamentous fungi: transcription and translation initiation and transcription termination sequences comprising nucleotide sequences depicted in Sequence Listing 1 from nucleotide 1 up to nucleotide 426 and from nucleotide 850 up to nucleotide 1514, respectively, or essentially the same nucleotide sequences, or functional parts thereof. Typically, these regulating sequences originate from gene Y. Also the use of the regulating sequences in a transformed host, particularly in a filamentous fungus host, preferably in P. chrysogenum, for the expression of a gene, by the fusion of the open reading frame of said gene to said regulating sequences have been provided for.

Finally, secretion signal encoding sequences comprising the nucleotide sequences depicted in Sequence Listing 1 from nucleotide 427 up to 537 or essentially the same nucleotide sequences, or functional parts thereof, and the use of these secretion signal encoding sequences, optionally together with the transcription and translation initiation sequences and/or termination sequences indicated above, in a transformed host, particularly in a filamentous fungus host, preferably in P. chrysogenum, for the secretion of a protein by the fusion of nucleotide sequences encoding and expressing said protein, to said secretion signal encoding sequences have been provided for.

### BRIEF DESCRIPTION OF THE FIGURES

- Figure 1: schematic representation of plasmid pRH01.
- Figure 2A: Northern blot analysis of gene Y expression; lane 1: P. chrysogenum Wisconsin 54-1255/lactose; lane 2: P. chrysogenum npe10/lactose; lanes 3-7: P. chrysogenum Wisconsin 54-1255 on saturating (lanes 3-4) and limiting (lanes 5-7) amounts of glucose.
- Figure 2B: SDS-PAAGE analysis of broth proteins; lane 1: molecular weight markers (LMW markers 94.000/67.000/43.000/30.000/20.100/14.400, Pharmacia, Sweden); lanes 2-3: P. chrysogenum Wisconsin 54-1255 broth from glucose (lane 3) and lactose (lane 2) cultures; lanes 4-5: P. chrysogenum npe10 broth from glucose (lane 5) and lactose (lane 4) cultures.

### BRIEF DESCRIPTION OF THE SEQUENCE LISTINGS

- Sequence Listing 1: nucleotide sequence of gene Y of P. chrysogenum and derived sequence of amino acids.
- Sequence Listing 2: amino acid sequence of protein Y of P. chrysogenum.
- Sequence Listing 3: partial nucleotide sequence of Y cDNA.
- Sequence Listing 4: partial N-terminal amino acid sequence of protein Y of P. chrysogenum.
- Sequence Listings 5-19: nucleotide sequences of gene Y derived synthetic oligonucleotides.

An efficient protein expression system is provided employing filamentous fungus species as hosts, particularly Penicillium chrysogenum. The expression system provides for DNA sequences for efficient initiation of transcription (promoter) and translation, DNA sequences for efficient termination of transcription and 3' mRNA processing (terminator), and DNA sequences necessary for efficient processing and secretion of the expressed protein.

The DNA sequences of the expression system were derived from gene Y (described in EP-A-354624) isolated from the filamentous fungus P. chrysogenum. Gene Y was highly expressed in P. chrysogenum and Y was efficiently secreted into the culture broth. Expression of a protein of interest is achieved by fusion of the open reading frame of the protein of interest to gene Y sequences. Fusions are made by using a variety of molecular genetic techniques which are well known in the art. The structure of the fusion genes can vary significantly depending on the site of fusion of the sequences encoding the protein of interest to gene Y sequences. By analogy to other expression systems it might be necessary to construct different fusion genes and to determine experimentally the optimal fusion construct for expression (M. Ward et al., Biotechnology 8 (1990), 435-440; and patent application WO 90/15860).

In a preferred embodiment of the invention the fusion gene encodes amino acid sequence: (see Sequence Listing 1) (one letter code), or functional parts thereof, as secretion signal. Fusion constructs are transformed in a filamentous fungus host, preferably P. chrysogenum, most preferably in a non-penicillin producing strain of P. chrysogenum, by methods known in the art. Transformants are selected by using a fungal selection marker like for instance the S. hindustanus phleomycin resistance gene (Drocourt et al., Nucl. Acids Res. 18 (1990), 4009), the A. nidulans amdS gene (Beri and Turner, Curr. Genet. 11 (1987), 693-641) or the niaD gene (Gouka et al., J. Biotechn. 20 (1991), 189-200) or facA gene (EP-A-487118) of P. chrysogenum. Transformants are purified and tested for expression of the protein of interest. Expression of gene Y was subject to glucose repression. Therefore, expression of proteins by using the gene Y expression system is controlled by the amount of glucose in the fermentation medium.

In another aspect of the invention gene Y promoter and terminator sequences are used for regulated, high-level expression of intracellular proteins by omitting the Y secretion signal sequence from the gene fusion. This application can be particularly useful to increase in a controlled manner the concentration of a protein, i.e. penicillin biosynthetic enzymes or proteins which do not occur naturally in P. chrysogenum, like cephalosporin of cephamycin biosynthetic enzymes.

In yet another aspect of the invention gene Y promoter sequences can be very useful as a tool in the isolation and identification of regulatory factors involved in regulation of penicillin biosynthetic genes which are also subject to glucose repression. Common regulatory factors can now be identified and separated from factors involved specifically in regulation of penicillin biosynthetic genes.

Although the gene Y expression system was obtained from P. chrysogenum, it can be applied to other filamentous fungus hosts like Penicillia, Acremonium, Aspergilli, Trichoderma and Mucor with well-known fermentation characteristics. Heterologous expression of promoters, terminators and secretion signals is a common observation in studies on gene expression in filamentous fungi. Alternatively, gene Y homologues can now easily be detected and isolated from other fungal species by using P. chrysogenum gene Y sequence as a probe or by using the Polymerase Chain Reaction (PCR) method and gene Y derived oligonucleotide probes.

The cloned gene Y of P. chrysogenum can also be used to create mutant Y genes with improved expression and/or secretion characteristics by using molecular genetic techniques well known in the art. It is also recognized that hybrid sequences for expression and secretion of proteins can be obtained by combining i.e. gene Y secretion signal sequences with other promoter or terminator sequences. Gene Y promoter, secretion signal and terminator sequences, or functional parts thereof, can be regarded and applied as individual cassettes in complete expression systems.

Moreover, the invention includes genes with different nucleotide sequences which are homologous to the Y gene of P. chrysogenum or parts thereof. Homology is defined herein as nucleotide sequences which have an identity score of at least 70% in a sequence comparison to gene Y by using the BestFit programme of the Wisconsin Sequence Analysis Software Package (version 6.0, release 1989, GCG, University of Wisconsin, USA), using parameter settings gap weight 5.000 and length weight 0.300. Homologous genes may be isolated from natural sources, or may be produced by mutagenesis of gene Y of P. chrysogenum.

In a preferred embodiment proteins are expressed in P. chrysogenum by using homologous gene Y expression signals.

The following examples are offered by way of illustration and not by way of limitation.

### EXAMPLES

Procedures in this application for gene cloning, gene characterization, gene manipulation and gene handling are well known in the art and adequately described in i.e. Sambrook et al. (Molecular Cloning, a Laboratory Manual, Cold Spring Harbor, USA, 1989).

The mutant strain npe10, obtained from P. chrysogenum Wisconsin 54-1255, has been deposited at the Centraal Bureau voor Schimmelcultures, Oosterstraat 1, 3742 SK Baarn, The Netherlands on March 13, 1990, under accession No. CBS 143.90.

### Example 1

### Gene Y

The isolation of gene Y, by using a differential screening method has been described in detail in EP-A-354624.

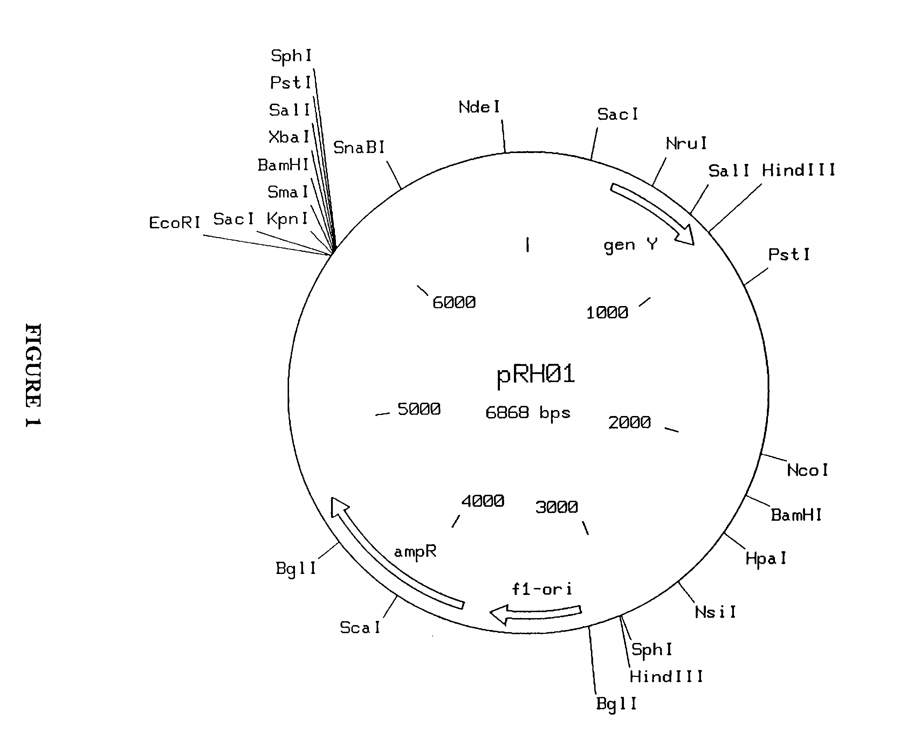

Gene Y was initially isolated on recombinant lambda phages B9, L5 and G5 (EP-A-354624) containing genomic DNA of P. chrysogenum and mapped to a 4 kilobase (kb) SpHI restriction fragment. The gene Y containing SphI restriction fragment of lambda G5 was subcloned into vector pTZ18R™ (Pharmacia, Sweden), in two orientations. The resulting plasmids have been named pRH01 and pRH02, respectively. Plasmid pRH01 is shown schematically in Figure 1. Plasmids pRH01 and pRH02 have been used to generate a more detailed restriction map of the 4 kb P. chrysogenum derived DNA (Figure 1) and to locate gene Y by hybridization analysis more precisely to a 1.2 kb SacI-HindIII restriction fragment near the centre of the 4 kb SphI fragment. Single stranded cDNA prepared as described in EP-A-354624 for differential screening was used as a probe. The direction of transcription of gene Y was also determined by hybridization analysis from SacI (5') to HindIII (3') restriction sites by using the same probe and single-stranded DNA derived from plasmids pRH01 and pRH02. Plasmids pRH01 and pRH02 were used to determine the nucleotide sequence of a 1.5 kb region encompassing the SacI-HindIII restriction fragment (Sequence Listing 1). The sequence analysis was performed according to procedures well known in the art (Sanger et al., Proc. Nat. Acad. Sci. USA 74 (1977) 5463-5467).

### Example 2

### Gene Y cDNA

Gene Y cDNA has been obtained by using a PCR strategy. PCR technology and other procedures for cDNA cloning, characterization, handling and manipulation are well known and adequately described in Sambrook et al. (supra) and Innis et al. (PCR Protocols, a Guide to Methods and Applications, Academic Press, 1990).

Total RNA was isolated from a penicillin producing culture of P. chrysogenum by using RNAzol™ (Cinna/Biotecx Lab. Int. Inc., Texas, USA), following the instructions of the manufacturers.

PolyA⁺ RNA was isolated from total RNA by oligo-dT cellulose chromatography using an mRNA purification kit (Pharmacia, Sweden). Approximately 60 µg of polyA⁺ RNA was isolated from approximately 200 µg of total RNA and dissolved into 200 µl of RNase free H₂O.

Synthesis of mRNA/cDNA hybrids was done by using Reverse Transcriptase and by using oligo-dT as a primer. A typical reaction contained: 17 µl of polyA⁺ mRNA, 1.9 µl of RNAsin (40 U/µl, Promega, USA), 10 µl of 5x RT buffer (250 mM Tris.HCl, pH 8.3, 15 mM MgCl₂, 375 mM KCl), 10 µl of 50 mM DTT (dithiothreitol), 5 µl of 8 mM dNTP-mix (dATP, dCTP, dTTP, dGTP at a concentration of 8 mM each), 5 µl of BSA (bovine serum albumin (1 mg/ml, Sigma, USA)), and 2.5 µl of Moloney Reverse Transcriptase (200 U/µl, BRL, FRG).

The reaction mixture was incubated at 37°C for 60 minutes with an extra aliquot of 1 µl of Reverse Transcriptase added after 30 minutes of incubation. The reaction was stopped by adding 50 µl of H₂O, 10 µl of 0.2 M EDTA and 110 µl of chloroform to the reaction mixture. Nucleic acids were purified by chloroform extraction and precipitation with ethanol. After centrifugation the pellet was resolved in 10 µl of H₂O giving an mRNA/cDNA hybrid concentration of approximately 0.5 mg/ml.

Oligonucleotides were chemically synthesized on a DNA synthesizer (Applied Biosystems, CA, USA). The sequence of the oligonucleotides, shown in Table I, is derived from the genomic sequence of gene Y, listed in Sequence Listing 1.

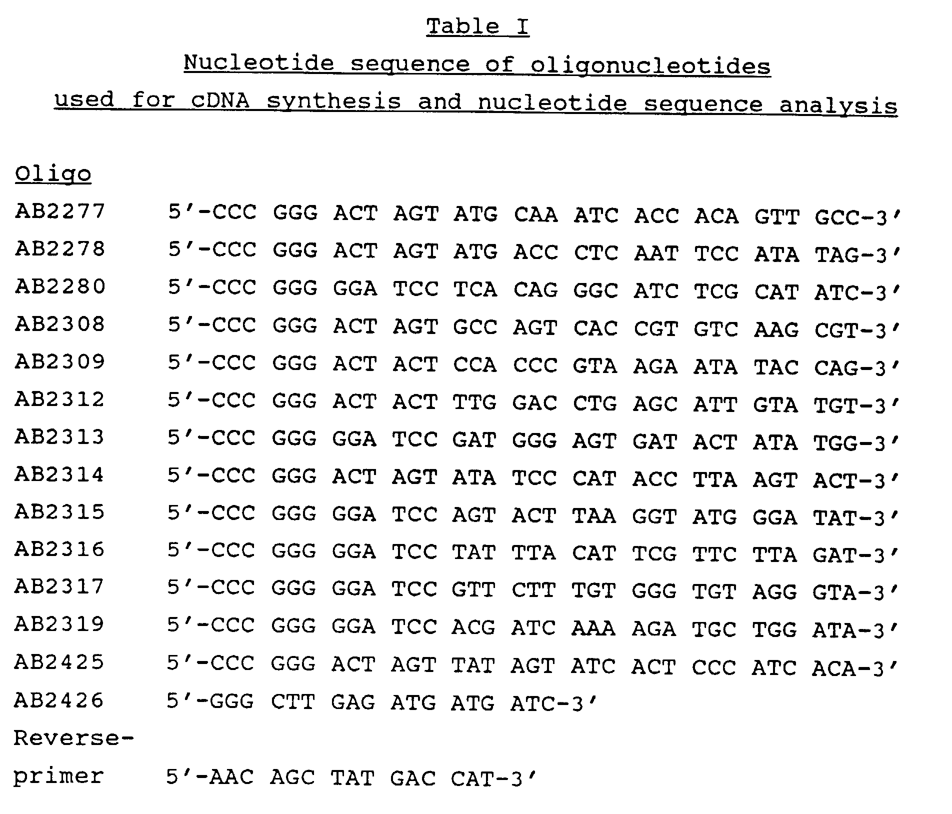

Oligonucleotides contained usually 21 nucleotides complementary to gene Y and a flanking sequence of 12 nucleotides containing SpeI and SmaI or BamHI and SmaI restriction enzyme recognition sites, respectively. Oligonucleotides were dissolved in 10 mM Tris.HCl, pH 8.0, 1 mM EDTA. Optimal concentrations for PCR were determined by electrophoresis on 4% Nu Sieve-GTG (FMC, USA) agarose gels in 1x TBE buffer (45 mM Tris.borate, 1 mM EDTA, pH 8.0; Sambrook, supra) and by PCR experiments.

PCR was used to isolate gene Y cDNA. A typical PCR reaction contained (50 µl volume): 5 µl of 10x R buffer (500 mM KCl, 100 mM Tris.HCl, pH 8.3, 20 mM MgCl₂, 0.1% (w/v) gelatin, 8 µl of a dNTP mixture (1.25 mM each of dATP, dCTP, dGTP and dTTP), optionally, 5 µl of DNA-dilution buffer (10 mM Tris.HCl, pH 8.0, 1 mM EDTA, 10 mM NaCl), approximately 0.05-0.1 µg of mRNA/cDNA hybrid, approximately 0.5 µg of oligonucleotide (each), and H₂O to make the final volume 50 µl.

PCR was performed by using 1.3 units of Taq DNA Polymerase and a PCR apparatus (DNA thermal cycler, Perkin Elmer Cetus, USA). Approximately 20-25 cycles were performed, depending on the length of the fragment (per cycle: 2 minutes 94°C; 2 minutes 55°C; 3 minutes 72°C, depending on the length of the fragment). In the last cycle the denaturation step was omitted. PCR products were analyzed by electrophoresis on 1.4% Nu Sieve agarose gels in 1x TBE buffer. PCR products were purified by extraction with chloroform, precipitation with ethanol and by using a RDP Mini column (Bio-Rad, CA, USA). PCR products were finally dissolved in H₂O, digested with restriction enzymes SpeI and BamHI, and ligated into SpeI, BamHI digested pBluescript II KS™ vector (Stratagene, CA, USA) following standard cloning procedures. E. coli strains HB101 (Boyer et al., J. Mol. Biol. 41 (1969), 459-465; and Sambrook et al., supra) or WK6 (Zell and Fritz, EMBO J. 6 (1987, 1809-1815) were used for transformation and plasmid propagation. The nucleotide sequence of cloned PCR fragments was determined by using double-stranded plasmid DNA or by isolating plasmid derived single-stranded DNA by super-infection of transformants with helper phage M13K07 (Vieira, J., Meth. Enzymol. 153 (1987), 3-11) and by application of several oligonucleotides, both pBluescript derived and gene Y derived, following procedures well known in the art (Sanger, supra).

The nucleotide sequence of gene Y cDNA, compiled from sequences of several different, overlapping PCR fragments is shown in Sequence Listing 3. The amino acid sequence deduced from the cDNA sequence is shown in Sequence Listing 1.

### Example 3

### Functional structure of gene Y

The functional structure of gene Y is summarized in Sequence Listing 1.

Nucleotides 1 to 1514 indicate the genomic sequence of gene Y. Nucleotides 553 to 628 and 719 to 786 indicate the position of two introns which disrupt the open reading frame (ORF). Two in-frame ATG start codons are found at positions 427-430 and 466-469, respectively, an UAG stop codon is found at position 847-849, which confines the ORF to nucleotides 427 to 849 or nucleotides 466 to 849, depending on the ATG codon used. The ORF identified encodes a small, cysteine-rich protein of 55 amino acids with an calculated molecular weight (MW) of 10013 D.

The N-terminal amino acid has a high content of hydrophobic amino acids typical for leader sequences involved in secretion.

Nucleotides 1 up to 426 define (part of) the region required for initiation of transcription (promoter), 5'-end processing of Y mRNA (non-translated leader), and initiation of translation, and is referred to as transcription and translation initiation regulating sequence. Within this sequence typical promoter elements have been recognized: a CAAT box at nucleotides 225-228 and a TATA box at nucleotides 312-315 followed by a pyrimidine-rich sequence.

The region required for termination of transcription and 3'-end processing of Y mRNA is confined to nucleotides 850 up to 1514, and is referred to as transcription termination regulating sequence.

### Example 4

### Expression of Y

P. chrysogenum strains were cultured on a standard complex medium (Revilla et al., J. of Bacteriol. 168 (1986), 947-952; and Revilla et al., J. Antibiot. 37 (1984), 781-789), containing either glucose or lactose as defined carbon source. After a period of 2-5 days total RNA was isolated from the mycelium as described in Example 2.

Samples, containing approximately 20 µg of total RNA were denatured by using glyoxal and DMSO (Sambrook, supra) and subjected to electrophoresis on 1.0% agarose gels in 10 mM phosphate buffer pH 7.0.

Following electrophoresis, RNA was blotted onto nylon membranes (GeneScreen⁺, New England Nuclear, USA) according to the instructions of the manufacturer. Northern blots were then probed with a mixture of two probes, both labelled by using a random priming labelling kit (Boehringer) and α[³²P] dATP (3000 Cu/mmol, Amersham, UK). Probe 1, the 4 kb SphI restriction fragment of plasmid pHR01 (Example 1), was used to detect y mRNA. Probe 2, the 1.2 kb HindII restriction fragment of plasmid pGJ02 (Veenstra et al., J. Biotechnol. 19 (1990), 81-90), was used to detect penDE mRNA. The latter probe generates by cross-hybridization a signal with 5S rRNA as well (Veenstra et al., In: 50 Years of Penicillin Application, Symposium in Honour of Sir Edward P. Abraham, September 8, 1990, Berlin, FRG) which is used as a control for the amount of RNA loaded. Standard, stringent, conditions for hybridization and washing of the membranes have been maintained (Sambrook, 1989, supra).

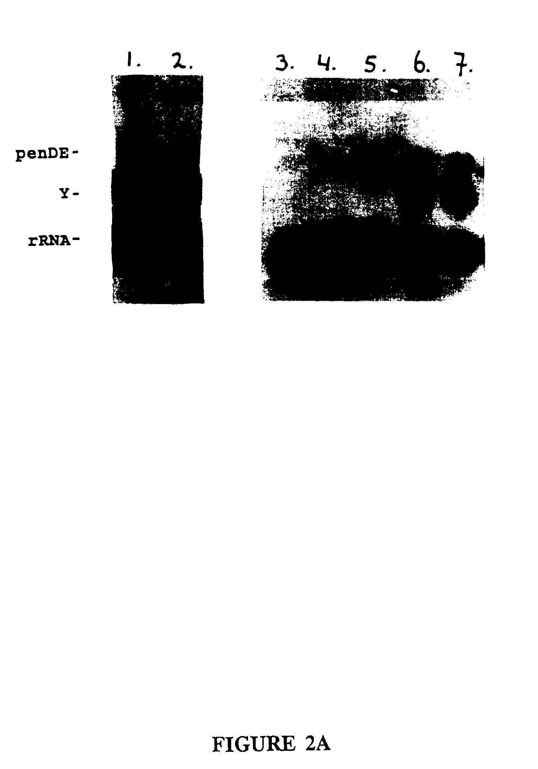

Typical results are shown in Figure 2A which demonstrates the high level of Y mRNA compared to penDE mRNA. Expression of gene Y is repressed by the presence of excess glucose and derepressed and/or induced by the absence of excess glucose.

Furthermore, efficient and regulated expression of gene Y is also observed in strain npe10, a non-penicillin derivative of P. chrysogenum Wisconsin 54-1255 lacking the pcbAB-pcbC-penDE gene cluster (EP-A-448180).

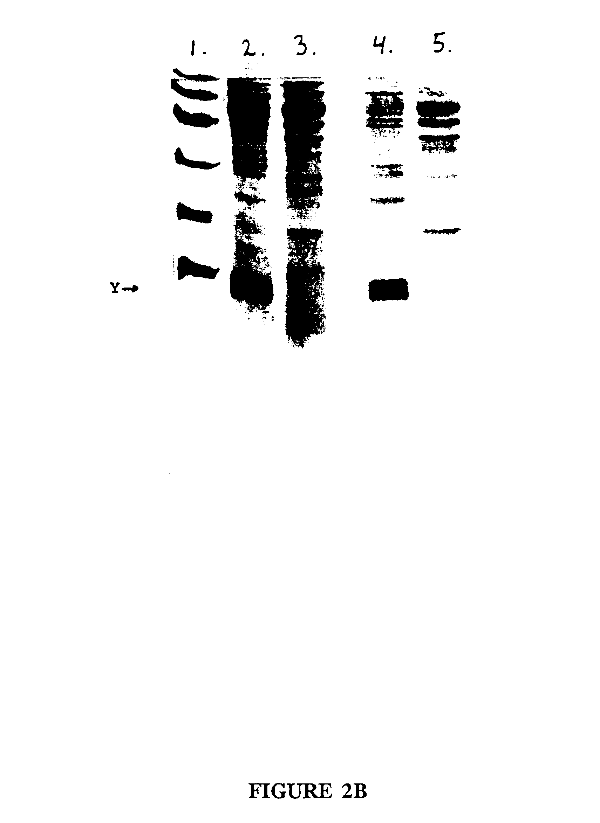

The expression of gene Y was also investigated at the protein level by analysis of proteins present in the broth of P. chrysogenum cultures. Broth samples were filtrated and concentrated by precipitation with methanol. The protein composition of the filtrate was analyzed on denaturing 17.5-20% PAAGE (acrylamide:bisacrylamide 39:1 w/w) 0.1% SDS slab gels, run in Tris.glycine-SDS buffer (25 mM Tris, 192 mM glycine, 0.1% SDS, pH 8.5) according to Laemlli (Nature 227 (1970), 680-685). Following electrophoresis proteins were stained in situ with Coomassie Brilliant Blue R250 and photographed. A typical result is shown in Figure 2B. It is clear from Figure 2B that a protein of the expected size of approximately 8 kD is highly expressed under conditions of Y mRNA expression. In addition to the strains shown in Figure 2B, P. chrysogenum strains containing multiple copies of gene Y, obtained by transformation of gene Y, showed an increased level of 8 kD protein in culture filtrates.

### Example 5

### Identification of Y

The identification of the 8 kD protein in culture filtrates of P. chrysogenum as the Y gene product was achieved by partial N-terminal amino acid sequence analysis. P. chrysogenum strain Wisconsin 54-1255 was grown as described in Example 4 under Y-expressing conditions (lactose as defined carbon source). Culture broth was collected, filtrated, concentrated and proteins were separated on denaturing SDS-PAAGE gels as described in Example 4. Following electrophoresis proteins were blotted onto a PVDF membrane (polyvinylidene difluoride, 0.45 µm pore size, Millipore, MA, USA) using a semi-dry blotting system (Nova blot, LKB, Sweden). After blotting proteins were visualized by staining with Coomassie Brilliant Blue R250. Membrane sections containing the 8 kD protein were collected and used for N-terminal sequence analysis by the Edman degradation procedure using an automatic analyzer (Applied Biosystems, USA). The amino acid sequence determined is shown in Sequence Listing 4. The sequence analysis proves the identity of the 8 kD protein as the Y gene product. Amino acids 1 to 21 of the mature secreted Y correspond to amino acids 38-58 of the ORF starting at the first ATG codon (see Example 3 and Sequence Listing 1). The leader sequence for processing and secretion of Y is hereby confined to the sequence of amino acids -37-1 listed in Sequence Listing 1.

### Example 6

### Phytase expression in Penicillium chrysogenum by using qene Y expression signals

A P. chrysogenum phytase expression cassette is constructed by fusion of sequences of the phytase gene of Aspergillus ficuum encoding the mature form of phytase (EP-A-420358) to expression signals of gene Y, i.e. the promoter, secretion signal and terminator sequences of gene Y. The fusion is made by application of the PCR method (Innis, supra) and results in the replacement of nucleotides 538 to 846 in Sequence Listing 1 with nucleotides 382 to 1715 of the phytase gene sequence published in Figure 6 of EP-A-420358. Plasmids pRH01 (Figure 1) and pAF2-2S (EP-A-420358) are used in PCR experiments. The resulting plasmid pPYPH01 contains the Y-phytase gene fusion as well as the S. hindustanus phleomycin resistance gene under control of the promoter of the phosphoglycerate kinase (PGK) gene of P. chrysogenum.

pPYPH01 is introduced into P. chrysogenum by transformation according to procedures well known in the art (Veenstra et al., supra). Transformants are selected for resistance to phleomycin, purified and then analyzed for expression of phytase by methods described in detail in EP-A-420358.

## Claims

1. A transcription and translation initiation regulating region originating from gene Y from Penicillium chrysogenum or a homologue thereof.

2. The region of claim 1 which extends up to nucleotide 426 of SEQ ID NO:1.

3. The region of claim 1 which extends up to nucleotide 537 of SEQ ID NO:1.

4. A transcription termination regulating region originating from gene Y from Penicillium chrysogenum or a homologue thereof.

5. A protein secretion signal encoding sequence comprising the nucleotide sequence depicted in SEQ ID NO:1 from nucleotide 427 up to nucleotide 537 or a homologue thereof.

6. A protein secretion signal encoding sequence encoding a peptide sequence comprising the amino acid sequence depicted in SEQ ID NO:2 from amino acid -37 up to amino acid -1 or a homologue thereof.

7. Use of the transcription and translation initiation region as defined in any one of claims 1 to 3 for expression of a desired protein in a transformed host, comprising fusion of said transcription and translation initiation region to the nucleotide sequence encoding said protein.

8. Use of the secretion signal encoding sequence as defined in claim 5 or 6 for the secretion of a desired protein by a transformed host, comprising fusion of said secretion signal encoding sequence to a nucleotide sequence encoding a mature form of said protein.

9. Use according to claim 7 or 8, wherein the transformed host is a filamentous fungus, preferably Penicillium chrysogenum.

10. A method for the production of a protein of interest, characterized in that a fungal host cell is transformed with an expression system comprising the open reading frame of the protein of interest having been fused to the transcription and translation initiation regulating region of claims 1 to 3, transformed host cells expressing the protein of interest are isolated and the thus-obtained cells are cultured under conditions conducive to expression of said protein of interest.

11. The method of claim 10, wherein the open reading frame of the protein of interest further has been provided with the transcription termination regulating region of claim 4.

## Patentansprüche

1. Transkriptions- und Translations-Initiationsregulierungsregion, stammend aus dem Gen Y von *Penicillium chrysogenum,* oder ein Homologes davon.

2. Region nach Anspruch 1, das sich bis zum Nucleotid 426 von SEQ ID NO:1 erstreckt.

3. Region nach Anspruch 1, das sich bis zum Nucleotid 537 von SEQ ID NO:1 erstreckt.

4. Transkriptions-Terminationsregulierungsregion, stammend aus dem Gen Y von *Penicillium chrysogenum,* oder ein Homologes davon.

5. Für ein Proteinsekretionssignal kodierende Sequenz, umfassend die in SEQ ID NO:1 dargestellte Nucleotidsequenz vom Nucleotid 427 bis zum Nucleotid 537 oder ein Homologes davon.

6. Proteinsekretionssignal-Kodierungssequenz, kodierend für eine Peptidsequenz, die die in SEQ ID NO:2 dargestellte Aminosäuresequenz von der Aminosäure -37 bis zur Aminosäure -1 umfaßt, oder ein Homologes davon.

7. Verwendung der Transkriptions- und Translationsinitiationsregion gemäß einem der Ansprüche 1 bis 3 zur Expression eines gewünschten Proteins in einem transformierten Wirt, umfassend die Fusion dieser Transkriptions- und Translations-Initiationsregion mit der für das Protein kodierenden Nucleotidsequenz.

8. Verwendung der Sekretionssignal-Kodierungssequenz gemäß Anspruch 5 oder 6 für die Sekretion eines gewünschten Proteins durch einen transformierten Wirt, umfassend die Fusion der Sekretionssignal-Kodierungssequenz mit einer Nucleotidsequenz, die für eine reife Form des Proteins kodiert.

9. Verwendung nach Anspruch 7 oder 8, wobei es sich beim transformierten Wirt um einen filamentösen Pilz, vorzugsweise *Penicillium chrysogenum,* handelt.

10. Verfahren zur Herstellung eines Proteins von Interesse, dadurch gekennzeichnet, daß eine Pilzwirtszelle mit einem Expressionssystem transformiert wird, das den offenen Leseraster des Proteins von Interesse, der mit der Transkriptions- und Translations-Initiationsregulierungsregion der Ansprüche 1 bis 3 fusioniert ist, die das Protein von Interesse exprimierenden transformierten Wirtszellen isoliert werden und die auf diese Weise erhaltenen Zellen unter Bedingungen, die zur Expression des Proteins von Interesse führen, gezüchtet werden.

11. Verfahren nach Anspruch 10, wobei der offene Leseraster des Proteins von Interesse ferner mit einer Transkriptions-Terminationsregulierungsregion nach Anspruch 4 versehen ist.

## Revendications

1. Région régulant l'initiation de la transcription et de la traduction provenant du gène Y de Penicillium chrysogenum ou homologue de celle-ci.

2. Région selon la revendication 1 qui s'étend jusqu'au nucléotide 426 de SEQ ID n° 1.

3. Région selon la revendication 1 qui s'étend jusqu'au nucléotide 537 de SEQ ID n° 1.

4. Région régulant la terminaison de la transcription provenant du gène Y de Penicillium chrysogenum ou homologue de celle-ci.

5. Séquence codant un signal de sécrétion de protéine comprenant la séquence nucléotidique décrite dans SEQ ID n° 1 depuis le nucléotide 427 jusqu'au nucléotide 537 ou homologue de celle-ci.

6. Séquence codant un signal de sécrétion de protéine codant une séquence peptidique comprenant la séquence d'acides aminés décrite dans SEQ ID n° 2 depuis l'acide aminé -37 jusqu'à l'acide aminé -1 ou homologue de celle-ci.

7. Utilisation de la région d'initiation de la transcription et de la traduction telle que définie dans l'une quelconque des revendications 1 à 3 pour l'expression d'une protéine voulue dans un hôte transformé, comprenant la fusion de ladite région d'initiation de la transcription et de la traduction avec la séquence nucléotidique codant ladite protéine.

8. Utilisation de la séquence codant un signal de sécrétion telle que définie dans la revendication 5 ou 6 pour la sécrétion d'une protéine voulue par un hôte transformé, comprenant la fusion de ladite séquence codant un signal de sécrétion avec une séquence nucléotidique codant une forme mature de ladite protéine.

9. Utilisation selon la revendication 7 ou 8, dans laquelle l'hôte transformé est un champignon filamenteux, de préférence Penicillium chrysogenum.

10. Une méthode pour produire une protéine d'intérêt, telle que des cellules hôtes fongiques soient transformées avec un système d'expression comprenant la région d' initiation de la transcription et de la traduction, telles que définiés dans l'une quelconque des revendications 1 à 3, fusionnée avec la séquence nucléotidique codant la dite protéine,
des cellules hôtes, ainsi transformées exprimant la protéine d'intérêt soient isolées et
ces cellules ainsi isolées soient cultivées sous conditions permettant l'expression de la dite protéine d'intérêt.

11. La méthode décrite dans la revendication 10, dans laquelle la séquence nucléotidique codant la protéine d'intérêt comprenne également la région régulant la terminaison de la transcription définié dans la revendication 4.


### SEQUENCE LISTING

(1) GENERAL INFORMATION:
   (i) APPLICANT: Gist-brocades N.V.
   (ii) TITLE OF INVENTION: A Eukaryotic Expression System
   (iii) NUMBER OF SEQUENCES: 19
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER:
(2) INFORMATION FOR SEQ ID NO:1:
   (i) SEQUENCE CHARACTERISTICS:
      (A) LENGTH: 1514 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (vi) ORIGINAL SOURCE:
      (B) STRAIN: Penicillium chrysogenum
   (ix) FEATURE:
      (A) NAME/KEY: exon
      (B) LOCATION: 427..552
   (ix) FEATURE:
      (A) NAME/KEY: intron
      (B) LOCATION: 553..628
   (ix) FEATURE:
      (A) NAME/KEY: exon
      (B) LOCATION: 629..718
   (ix) FEATURE:
      (A) NAME/KEY: intron
      (B) LOCATION: 719..786
   (ix) FEATURE:
      (A) NAME/KEY: exon
      (B) LOCATION: 787..846
   (ix) FEATURE:
      (A) NAME/KEY: CDS
      (B) LOCATION: join(427..552, 629..718, 787..846)
      (D) OTHER INFORMATION: /codon_start= 427 /product= "Antifungal protein (preprotein)"
   (ix) FEATURE:
      (A) NAME/KEY: sig_peptide
      (B) LOCATION: 427..537
   (ix) FEATURE:
      (A) NAME/KEY: mat_peptide
      (B) LOCATION: 538..846
      (C) IDENTIFICATION METHOD: experimental
      (D) OTHER INFORMATION: /product= "Antifungal protein" /evidence= EXPERIMENTAL
   (xi) SEQUENCE DESCRIPTION: SEQ ID NO:1:
   GCCAGTCACC GTGTCAAGCG TTCAGCCGTT TCTCTGCTTT TTAGGAAATT GATTACCACT    60
   AGGTAAGCCC AAAAATATCT TCCTGGTAAA CAAGTAGTGC ATTCTTACCC CGGAGGCTGA   120
   AGCAGGTAAG GGATTTTGGA GAGACCCCAC CCGTAAGAAT ATACCAGCCA AGAGGTCCAG   180
   TATCCTGAAG TATGTGAGGC ATTAATGTCA TTGGAGAAGT CATGCAATCC ATAAGCTGCC   240
   ACCCCCAAGA TGACTGCATT GGACCTGAGC ATTGTATGTG TCACCTTTCA CACAGAGCTC   300
   ATGATCTGGT TTATAAAGGC GGCTTCATGA CCCTCAATTC CATATAGTAT CACTCCCATC   360
   ACAGCATTTC GATATCTTCA ACCACTTTAA CCTTCTCCAG AGGATCATCA TCTCAAGCCC   420
   TTCATA ATG CAA ATC ACC ACA GTT GCC CTT TTT CTC TTC GCT GCA ATG      468
          Met Gln Ile Thr Thr Val Ala Leu Phe Leu Phe Ala Ala Met
          -37     -35                     -30                 -25
   GGC GGG GTA GCC ACC CCC ATT GAG TCT GTA TCA AAC GAC CTC GAT GCC     516
   Gly Gly Val Ala Thr Pro Ile Glu Ser Val Ser Asn Asp Leu Asp Ala
               -20                 -15                 -10
   AGG GCT GAG GCC GGT GTC CTG GCC AAA TAC ACC GGA GTGAGTAAAC          562
   Arg Ala Glu Ala Gly Val Leu Ala Lys Tyr Thr Gly
            -5                 1               5
   ATCAATATCC CATACCTTAA GTACTCACTT GGGAATCGCG ACTAACGGTT CGGGACCACA   622
   ACTCAG AAA TGC ACC AAA TCT AAG AAC GAA TGT AAA TAC AAG AAC GAT      670
          Lys Cys Thr Lys Ser Lys Asn Glu Cys Lys Tyr Lys Asn Asp
                           10                  15
   GCT GGA AAG GAC ACT TTT ATC AAG TGC CCC AAG TTT GAT AAC AAG AAG     718
   Ala Gly Lys Asp Thr Phe Ile Lys Cys Pro Lys Phe Asp Asn Lys Lys
    20                  25                  30                  35
   GTAGAATATC AATCATTCGG AAGTAGCCAT CTGAATCGAT TTCGTGCTAA TCTCGCTCTT   778
   TTTTCCAG TGC ACC AAG GAT AAT AAC AAA TGT ACC GTC GAC ACC TAC AAC      828
            Cys Thr Lys Asp Asn Asn Lys Cys Thr Val Asp Thr Tyr Asn
                            40                  45
   AAC GCT GTC GAT TGT GAC TAGATGGTCT CTGCGATCAC CAGGGCATTT              876
   Asn Ala Val Asp Cys Asp
    50                 55
   AATGGTTTTT GGTTCCCTTC TTGTTGGTGA TATGCGAGAT GCCCTGTGAT TCTCGAAGCT     936
   TACTACCCTA CACCCACAAG GAACTCGGAA CCAAGGAACT GCTCGGTGGG TGATACATAT     996
   ACACCCAGTA TCTATCCAGC TTCAATTTTC GGCGAATTTT GTTTCTTATT TCATAAAGAC    1056
   ACTCGTTTGA TATCTAGCTA GATATTGTTG CTCATCAACG AAATGGTTGT AGATTATCGA    1116
   ATATATCCAG CATCTTTTGA TCGTAGTCGG AAGTGAAATG GAGTACTATG ATACGACACA    1176
   TGTACATTGT AAGCAGAAAT AGGCTAGAGG GATAACTATC AAACTGCTGC AGCAGCGCTA    1236
   CTCTTGCTTC TGTGCGGGGT CAAACTTGTT TGCGAGCCGG ACTGCCAAAT CAGGGTCTGG    1296
   GTAATGCTGG GGGCCGATTC CCTGTTATGC GGTAAGGATT AACTGGGGTT CTCAAAATGT    1356
   TTCACACAGC CACTTCGTTA TTCCTTATAC CTGCCAAAAT CCCGCAATTT AATTCCTTAG    1416
   TACACCCGTT ATACATCTAT CGCTGATAAG GTTTATCATA GGTACAATAG CTTTGATTAT    1476
   AGGGACACGT CCAATGCTTA AATGCAATTT CCTTAACA                            1514
(2) INFORMATION FOR SEQ ID NO:2:
   (i) SEQUENCE CHARACTERISTICS:
      (A) LENGTH: 92 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gln Ile Thr Thr Val Ala Leu Phe Leu Phe Ala Ala Met Gly Gly
   -37     -35                 -30                 -25
   Val Ala Thr Pro Ile Glu Ser Val Ser Asn Asp Leu Asp Ala Arg Ala
       -20                 -15                 -10
   Glu Ala Gly Val Leu Ala Lys Tyr Thr Gly Lys Cys Thr Lys Ser Lys
    -5                     1               5                   10
   Asn Glu Cys Lys Tyr Lys Asn Asp Ala Gly Lys Asp Thr Phe Ile Lys
               15                  20                  25
   Cys Pro Lys Phe Asp Asn Lys Lys Cys Thr Lys Asp Asn Asn Lys Cys
           30                  35                  40
   Thr Val Asp Thr Tyr Asn Asn Ala Val Asp Cys Asp
       45                  50                  55
(2) INFORMATION FOR SEQ ID NO:3:
   (i) SEQUENCE CHARACTERISTICS:
      (A) LENGTH: 355 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (vi) ORIGINAL SOURCE:
      (A) ORGANISM: Penicillium chrysogenum
   (xi) SEQUENCE DESCRIPTION : SEQ ID NO:3:
   ATGCAAATCA CCACAGTTGC CCTTTTTCTC TTCGCTGCAA TGGGCGGGGT AGCCACCCCC    60
   ATTGAGTCTG TATCAAACGA CCTCGATGCC AGGGCTGAGG CCGGTGTCCT GGCCAAATAC   120
   ACCGGAAAAT GCACCAAATC TAAGAACGAA TGTAAATACA AGAACGATGC TGGAAAGGAC   180
   ACTTTTATCA AGTGCCCCAA GTTTGATAAC AAGAAGTGCA CCAAGGATAA TAACAAATGT   240
   ACCGTCGACA CCTACAACAA CGCTGTCGAT TGTGACTAGA TGGTCTCTGC GATCACCAGG   300
   GCATTTAATG GTTTTTGGTT CCCTTCTTGT TGGTGATATG CGAGATGCCC TGTGA        355
(2) INFORMATION FOR SEQ ID NO:4:
   (i) SEQUENCE CHARACTERISTICS:
      (A) LENGTH: 21 amino acids
      (B) TYPE: amino acid
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Penicillium chrysogenum
   (xi) SEQUENCE DESCRIPTION: SEQ ID NO:4:
   Ala Lys Tyr Thr Gly Lys Xaa Thr Lys Ser Lys Asn Glu Xaa Lys Tyr
   1               5                   10                  15
   Lys Asn Asp Ala Gly
               20
(2) INFORMATION FOR SEQ ID NO:5:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE : DNA (synthetic)
   (iii) HYPOTHETICAL: NO
   (vi) ORIGINAL SOURCE:
      (C) INDIVIDUAL ISOLATE: AB2277
   (xi) SEQUENCE DESCRIPTION : SEQ ID NO:5:
   CCCGGGACTA GTATGCAAAT CACCACAGTT GCC                    33
(2) INFORMATION FOR SEQ ID NO:6:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2278
   (xi) SEQUENCE DESCRIPTION: SEQ ID NO:6:
   CCCGGGACTA GTATGACCCT CAATTCCATA TAG                    33
(2) INFORMATION FOR SEQ ID NO:7:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2280
   (xi) SEQUENCE DESCRIPTION: SEQ ID NO:7:
   CCCGGGGGAT CCTCACAGGG CATCTCGCAT ATC 33
(2) INFORMATION FOR SEQ ID NO:8:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2308
   (xi) SEQUENCE DESCRIPTION: SEQ ID NO:8:
   CCCGGGACTA GTGCCAGTCA CCGTGTCAAG CGT 33
(2) INFORMATION FOR SEQ ID NO:9:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2309
   (xi) SEQUENCE DESCRIPTION: SEQ ID NO:9:
   CCCGGGACTA GTCCACCCGT AAGAATATAC CAG 33
(2) INFORMATION FOR SEQ ID NO:10:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2312
   (xi) SEQUENCE DESCRIPTION: SEQ ID NO:10:
   CCCGGGACTA GTTTGGACCT GAGCATTGTA TGT 33
(2) INFORMATION FOR SEQ ID NO:11:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2313
   (xi) SEQUENCE DESCRIPTION: SEQ ID NO:11:
   CCCGGGGGAT CCGATGGGAG TGATACTATA TGG 33
(2) INFORMATION FOR SEQ ID NO:12:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vii) IMMEDIATE SOURCE:
      (B) CLONE: AB2314
   (xi) SEQUENCE DESCRIPTION: SEQ ID NO:12:
   CCCGGGACTA GTATATCCCA TACCTTAAGT ACT 33
(2) INFORMATION FOR SEQ ID NO:13:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2315
   (xi) SEQUENCE DESCRIPTION: SEQ ID NO:13:
   CCCGGGGGAT CCAGTACTTA AGGTATGGGA TAT 33
(2) INFORMATION FOR SEQ ID NO:14:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2316
   (xi) SEQUENCE DESCRIPTION: SEQ ID NO:14:
   CCCGGGGGAT CCTATTTACA TTCGTTCTTA GAT 33
(2) INFORMATION FOR SEQ ID NO:15:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2317
   (xi) SEQUENCE DESCRIPTION: SEQ ID NO:15:
   CCCGGGGGAT CCGTTCCTTG TGGGTGTAGG GTA 33
(2) INFORMATION FOR SEQ ID NO:16:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2319
   (xi) SEQUENCE DESCRIPTION : SEQ ID NO:16:
   CCCGGGGGAT CCACGATCAA AAGATGCTGG ATA 33
(2) INFORMATION FOR SEQ ID NO:17:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2425
   (xi) SEQUENCE DESCRIPTION: SEQ ID NO:17:
   CCCGGGACTA GTTATAGTAT CACTCCCATC ACA 33
(2) INFORMATION FOR SEQ ID NO:18:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: AB2426
   (xi) SEQUENCE DESCRIPTION: SEQ ID NO:18:
   GGGCTTGAGA TGATGATC 18
(2) INFORMATION FOR SEQ ID NO:19:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: reverse sequencing primer
   (xi) SEQUENCE DESCRIPTION: SEQ ID NO:19:
   AACAGCTATG ACCATG 16